# EUROPEAN PATENT APPLICATION

(11) **EP 4 814 480 A1**
(43) Date of publication of application: **30.09.2026**
(21) Application number: 26167546.6
(22) Date of filing: 25.03.2026
(51) Int. Cl.: G01N 23/00, G01N 33/00, G01T 1/167, G01T 1/20

(54) **SCINTILLATION DETECTOR FOR RADIONUCLIDES AND RELATED METHOD OF DETECTION**

(30) Priority: 26.03.2025 IT 202500006228
(71) Applicant: NG Detectors S.r.l., 00198 Roma (IT)
(72) Inventor: PERGOLA, Andrea, 00198 Roma (IT); PANI, Roberto, 00198 Roma (IT)
(74) Representative: Leone, Mario

(57) **Abstract**

A detecting apparatus (10) for identifying and monitoring ionizing radiations, which can be used in situ for environmental detections, is provided with a detecting device (1) which comprises: a shielding shell (2) provided with a movable sealing lid; a scintillation crystal (4), contained in the inner space of the shielding shell (2), with opposed and separated receiving base (4A) and transmission base (4B); a fixed volume container (3), to receive a sample to be analysed, contained in said shielding shell (2) in a position adjacent to the receiving base (4A) and at the top opening (22B) of the device; a photomultiplier (5) extended on a geometrical plane adjacent to said transmission base (4B); and a spacer cage (6) inserted in an empty space (2A) of the shielding shell (2) which contains the scintillation crystal (4) surrounding it laterally so as to keep it aligned with the photomultiplier.

## Description

The present invention generally relates to a detecting apparatus of the scintillation type, for monitoring ionizing radiations, in particular of the type emitted by radionuclides dispersed in the environment, and to a method for monitoring the ionizing radiations with said detecting apparatus.

In recent decades, there has been an increasing need for detecting, quantifying and monitoring the contamination of sites where radioactive material leaks and spills occurred, for example due to accidents, accidental release of waste, environmental pollution and accumulation of radioactive substances in the environment.

To date, these environmental monitoring activities are carried out by taking samples from the environment itself, such as air, soil, water, food, plants or even objects; the collected material is transported to specialized laboratories which are equipped with complex and bulky equipment that substantially can be used only by specialized operators.

Alternatively, portable radiometers are used which detect radiation dose levels or portable spectrometers capable of identifying the presence of radionuclides in the environment, but which are not capable of quantifying them with the required precision to establish alert or health emergency levels in a short time and on site, so as to be able to evaluate, already during the inspection phase, whether to intervene and with which countermeasures, and without involving a laboratory.

Chinese patent No. CN 111323806 B describes a system for detecting radiations in the gases comprising a volume shielded by a container wherein a scintillation crystal is provided, surrounded by an empty volume wherein air and/or gas to be analysed is made to circulate. In the system, the scintillation crystal is constrained on the upper side and on the lower side, being inserted into two recesses.

Chinese patent No. CN 111323806 B describes a movable device for detecting radiations which comprises a volume shielded by a container, wherein a scintillation crystal is provided, inserted, for the most part, in a shielded chamber and, for a smaller portion of the upper part, inside a Marinelli Baker implemented so as to constrain the position of the crystal in conjunction with a gasket placed at the entrance of said shielded chamber.

In particular, it is noted that the measurements of gamma emissions in air are very critical, considering the variability resulting from winds or rain, but they are predictive for the fallout on the soil where the radionuclides will gradually accumulate, causing gamma emissions at first on vegetables and then on animals with much higher levels.

In case of emergency, the speed of measurement is fundamental for health interventions on the population, and it is necessary to proceed immediately with the identification of the isotopes present with a sufficiently low minimum detectable activity (MDA), compatible with the levels of health attention.

It is clear that this does not exclude that the sample is subsequently examined under laboratory conditions, with measurement time of 12-24 hours, by obtaining a complete analysis of the present isotopes with MDA values even 100 times lower, but incompatible with health interventions which have to take place within few hours from the dispersion of the radionuclides in the environment.

Moreover, it has to be considered that the environmental radiation background naturally present in an area of interest, which in Italy varies on average from 60 nSv/h to 240 nSv/h, influences MDA for statistical reasons, in particular for rapid measurements, by at least a factor of two in presence of adequate shielding, and much more in absence of shielding.

The weight of shielding of a detecting device, which is essential to make the device itself portable, depends upon its thickness, which is essential to reduce the environmental background, and upon the free internal volume, available to the sample, of a scintillation crystal and the related detection electronics.

Since the sample and crystal volumes cannot be minimized, on the contrary it is possible to minimize the electronics volume by maximizing the thickness of the lead shielding, it is then possible to minimize the minimum detectable activity (MDA) even under conditions of high radiation background.

Therefore, the portability is essential, considering the unpredictability of the area of interest.

The technical problem underlying the present invention is to provide a detecting apparatus for monitoring the radiations, a related kit and a method which provides the application of said detecting apparatus, allowing to obviate the drawback mentioned with reference to the known art, by implementing a device which, thanks to its own structure, can be constructed in a portable version.

Such problem is solved by a detecting apparatus and by a method as specified above and as respectively defined in the respective enclosed independent claims.

The detecting apparatus for identifying and monitoring radiations according to the invention is characterized in particular in that it comprises a detecting device in turn comprising a shielding shell for ionizing radiations, implemented by way of example with lead walls having adequate thickness, formed by a base and by a shield which are mechanically coupled so as to form an inner space of the device and a top opening thereof opposite the base.

Such shielding shell is provided with a sealing lid which is movable between an open position and a closed position wherein the top opening is hermetically sealed.

The apparatus further comprises a scintillation crystal which is housed in said inner space of the shell, arranged in such a way to extend along the longitudinal symmetry axis of the device, which defines, along with the cylindrical or parallelepiped shape thereof, a receiving base and a transmission base opposed and separated by at least one lateral wall.

Moreover, a fixed volume container is provided, arranged to receive a sample to be analysed, which is contained in said shielding shell in a position adjacent to the receiving base of the scintillation crystal and at said top opening therefrom it is accessible and can be extracted.

The detecting device also comprises a photomultiplier substantially extending on a geometrical plane adjacent to said transmission base of the scintillation crystal, that is between the crystal and the base of the shielding shell; the photomultiplier is provided with an electrical connection.

In the shell a spacer cage is provided, inserted in an empty space of said shielding shell containing the scintillation crystal surrounding its side wall(s) so as to act as spacer frame which is capable of keeping always aligned the scintillation crystal with the silicon photomultiplier, which in turn is centred with respect to said axis of symmetry and lastly spaced apart from the internal surface of the shield.

Said spacer cage defines an external geometrical surface arranged to match with said internal surface of the shield and an internal geometrical surface arranged to match with said lateral wall, thereby said spacer cage and said scintillation crystal are stuck in said inner space.

In other words, the external and internal geometrical surfaces are respectively complementary to the internal surface of the shield and to the lateral wall, being arranged to cooperate with each other by forming a fixed positional coupling.

Lastly, in the detecting device the shielding shell, between shield and base, has a recess implementing a passage channel for said electrical connection.

The main advantage of the detecting apparatus according to the present invention lies in the fact that said detecting device is wholly shielded by said shell, allowing to detect in situ very low radioactivity levels and the identification of the emitting radionuclides. Under very low radioactivity levels the ones having the capability of detecting a Minimum Detectable Activity (MDA) starting from 2 Bq depending on the type of isotope and measurement time are meant.

In fact, the lead shielding shell, by shielding said wall of said crystal and said container containing a sample to be analysed, minimizes the effects of radiation scattering by the surrounding world.

Another advantage of the detecting apparatus lies in an optimization of the overall volume with respect to the useful space to contain the sample in said fixed volume container for a sample to be analysed. In fact, having a fixed emitter sample volume allows to size said scintillation crystal to optimize the absolute detection efficiency of said detecting device with minimum volumes, allowing to reduce even the shielding volume.

According to a common inventive concept, the kit for identifying and monitoring ionizing radiations comprises a detecting apparatus as described in the claims.

The kit further comprises two or more scintillation crystals with different sizes from each other, two or more spacer cages with different thicknesses from each other.

Said two or more spacer cages define an external geometrical surface equal for all of them and an internal geometrical surface different for each one of them.

Preferably, said scintillation crystals and said spacer cages are combined to each other in a fixed positional relation, to keep aligned each scintillation crystal with the silicon photomultiplier, centred with respect to said axis of symmetry and spaced apart from the internal surface of the shield.

Preferably, said external geometrical surface is arranged to match with said internal surface of the shield, and said internal geometrical surface of each spacer cage is arranged to match with said lateral wall of the respective scintillation crystal, thereby said spacer cage and said scintillation crystal are stuck in said inner space.

Advantageously, the fact of having available scintillation crystals with different sizes and having respective cages associated to each crystal allows to replace a scintillation crystal with another one depending upon the crystal type, the analysed material and the volume of the analysed material. In this way the crystals with the respective cages are interchangeable and the detecting apparatus becomes adaptable without having to be subjected to modifications of said shielding shell.

According to a common inventive concept, the method for monitoring the type and concentration of radionuclides in a fixed volume sample comprises the use of a detecting apparatus of ionizing radiations as defined above, and provides the steps of:
- acquiring gamma radiations generated by the environmental background;
- performing a self-calibration of the detecting apparatus through software implemented on said processing unit; and
- simultaneously with self-calibration, arranging a sample to be analysed in a container;
- arranging said container in said detecting apparatus;
- identifying the radionuclides which generate an emissive gamma activity in the sample;
- performing the measurement of the emissive gamma activity of the sample; and
- transmitting the obtained data to an electronic device to make them visible to an operator.

The main advantage of the method according to the present invention lies in the fact of optimizing the time for identifying and monitoring the radiations from radionuclides; in fact, being able to perform calibration concurrently with the sample collection reduces the method execution time.

An additional advantage of the method lies in the fact of being able to perform the identification and monitoring of the radiations from radionuclides *in situ,* that is in the place where the sample is taken, without having to transport the sample in a dedicated laboratory, by optimizing the monitoring time and efficiency.

The present invention will be described hereinafter according to a preferred embodiment example thereof, provided by way of example and not for non-limiting purposes with reference to the enclosed drawings wherein:
* figure 1 shows a section lateral view of a detecting device according to the invention;
* figure 2 shows an exploded view of the detecting device of figure 1;
* figure 3 shows a front view of a detecting apparatus according to the invention;
* figure 4A shows a perspective view of the detecting apparatus of figure 3 with a lid according to the invention in open position and without fixed volume container for a sample to be analysed; and
* figure 4B shows a perspective view of the detecting apparatus of figure 3 with the lid in open position and with a fixed volume container for a sample to be analysed.

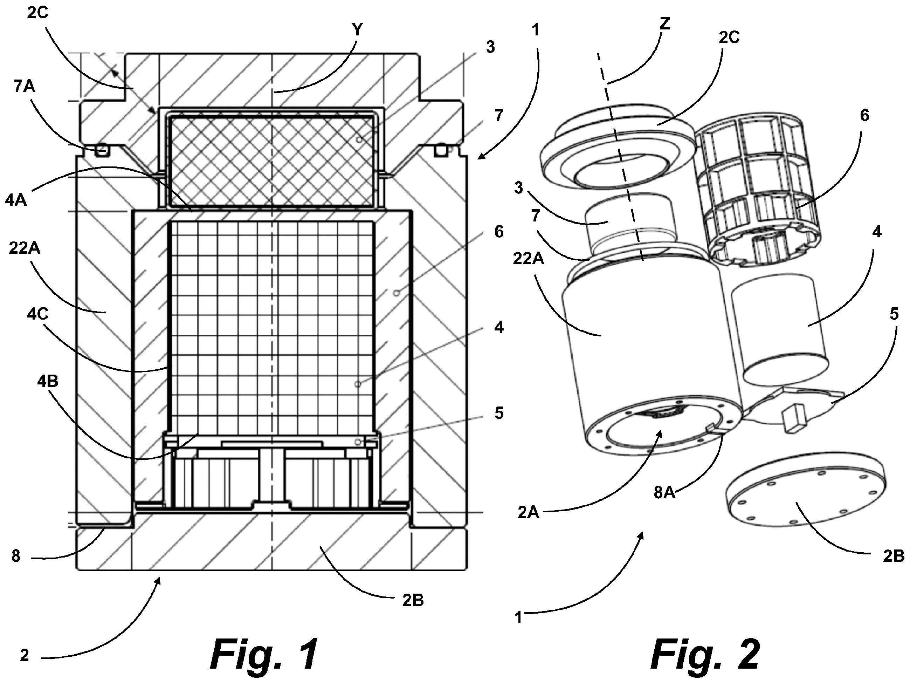

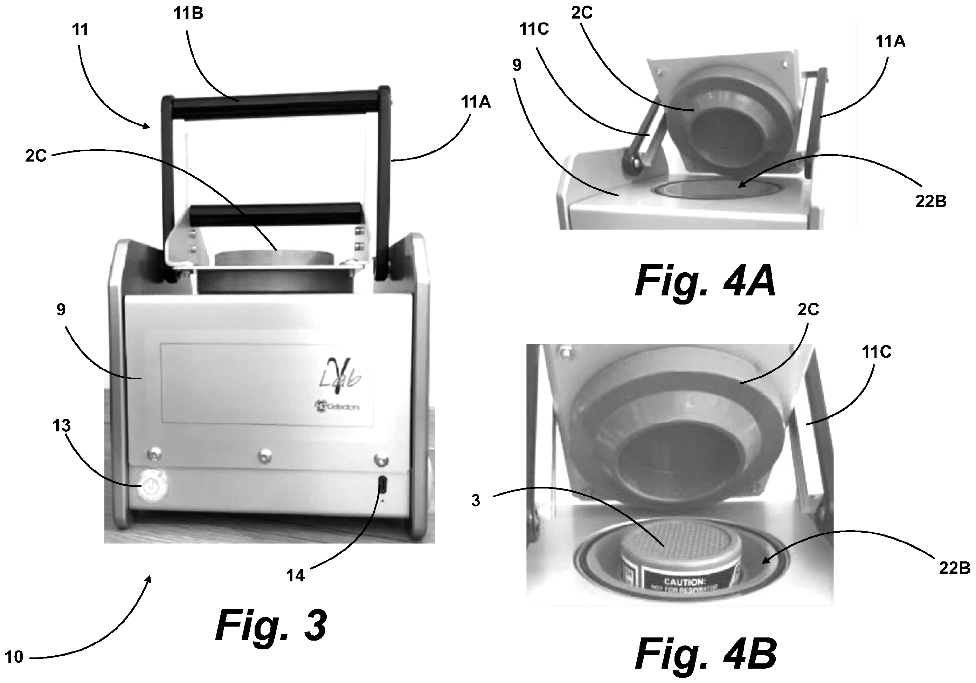

With reference to figures, and in particular to figure 3, a detecting apparatus 10 for identifying and monitoring radiations from radionuclides comprises a detecting device 1 as visible in figure 1 and figure 2.

The detecting device 1 comprises a lead shielding shell 2 which comprises an empty space 2A defined by a shield 22A and by a base 2B mechanically couplable to each other, with a top opening 22B opposite the base 2B.

The shield 22A comprises a recess 8A arranged to form a passage channel 8 for an electrical connection through the shielding shell 2.

The shielding shell 2 further comprises a lid 2C which is movable between an open position and a closed position, in which it remains preferably by simple gravity.

Alternatively, the lid 2C can be kept in the closed position by means of a conventional closing mechanism.

Preferably, the shielding shell 2 has a thickness comprised between 15 mm and 30 mm, preferably 15 mm.

Preferably, the detecting device 1 comprises a gasket 7 arranged between the upper edge of the shield 22A and said lid 2C when the latter is in a closed position.

With reference to figure 1, the detecting device 1 comprises a groove 7A in an upper portion 22B of the shield 22A, wherein said upper portion 22B is in contact with said lid 2C, when the latter is in closed position; the groove 7A is arranged to house the gasket 7.

Preferably, the gasket 7 has a larger cross section than the cross section of the groove 7A, and it is housed in the latter by elastic deformation. In these cases, when the lid 2C is in closing position, it exerts a force to elastically deform the gasket 7, i.e. to seal hermetically, that is to insulate a detection volume inside said detecting device 1 when said lid 2C is in closing position.

With reference to figures 1 and 4B, the detecting device 1 comprises a fixed volume container 3 to receive inside thereof a sample to be analysed.

In this example, the container 3 has cylindrical shape and it can have a diameter comprised between 40 mm and 70 mm, preferably it has a diameter of 50 mm or 58 mm, and a height comprised between 1 mm and 40 mm, preferably 1 mm or 27 mm.

According to some preferred embodiments, the container 3 has a diameter of 50 mm and a height of 1 mm.

According to other preferred embodiments, the container 3 has a diameter of 58 mm and a height of 27 mm.

Preferably, the sample is a sample of soil, water, air or a combination thereof.

Preferably, the sample is a sample of air.

According to other embodiments of the invention not shown in figures, the container 3 comprises a first cellulose filter and, preferably, a second active carbon filter and it is arranged to contain air samples.

The detecting device 1 further comprises a scintillation crystal 4 extending along a longitudinal symmetry axis Y of the detecting device 1. The crystal 4 has a shape so as to define opposed receiving base 4A and transmission base 4B, separated by at least one continuous lateral wall 4C, as in case of a cylindrical shape, or by several lateral walls as in case of a parallelepiped shape.

In contact with said transmission base 4B, that is in the space comprised between it and the base 2B of the shielding shell 2, the detecting device 1 comprises a photomultiplier 5, in particular a silicon photomultiplier (SiPM), substantially extended on a geometrical plane defined between base 2B and transmission base 4B.

Advantageously, said fixed volume container 3 is in contact with said receiving base 4B. Moreover, said container 3 can have a source symmetry axis Z coincident with said longitudinal symmetry axis Y of the scintillation crystal 4 and with a centre of said SiPM 5.

It will be appreciated that the sample to be analysed is also called source and that the front radiation condition of the scintillation crystal 4 (coinciding source symmetry axis Z and longitudinal symmetry axis Y) represents the ideal condition in defining a fixed solid angle and to obtain a spectrum (response function of the apparatus 10 for detecting radiations from radionuclides) not influenced by the radiation position.

Moreover, the lead shielding of the shell, surrounding the scintillation crystal 4 and the fixed volume container 3, minimizes the effects of scattering of the radiation by the same detecting apparatus 10, and then possible over-evaluations of incident radiation.

To allow the alignment of the source symmetry axis Z with the longitudinal symmetry axis Y, the detecting device 1 comprises a spacer cage 6.

Moreover, the cage 6 surrounds the wall 4C of the scintillation crystal 4 thereby aligning said longitudinal symmetry axis Y even with the centre of said SiPM 5.

The cage 6 comprises a thickness substantially corresponding to the distance between the wall 4C and the shield 22A, said thickness is preferably comprised between 5 mm and 30 mm, more preferably 10 mm.

Advantageously, a cage 6 can be implemented with different thicknesses, so as to make possible the use of scintillation crystals 4 with different sizes of a section transversal to the longitudinal symmetry axis Y, without having to modify the sizes of a section transversal to the longitudinal symmetry axis Y of the shielding shell 2 and/or the thickness thereof.

In fact, the volume of the sample to be analysed being equal, when the type of scintillation crystal 4 varies it could be necessary or preferred to vary the sizes of the cross section thereof to obtain the same efficiency in analysing the radiations from radionuclides, independently from the used scintillation crystal 4. Should it be possible and/or necessary to reduce the sizes of the section transversal to the longitudinal symmetry axis Y of the scintillation crystal 4, it would be possible to implement a cage 6 with larger thickness meanwhile keeping said longitudinal symmetry axis Y aligned, even with the centre of said SiPM 5.

Moreover, the presence of the cage 6 also allows to protect the integrity of the scintillation crystal 4 which could be damaged due to rubbing between the wall 4C and the shield 22A.

According to some preferred embodiments not shown in figures, the invention comprises a kit for identifying and monitoring ionizing radiations comprising a detecting apparatus 10 as described in the claims. The kit further comprises two or more scintillation crystals 4 with different sizes from each other and two or more spacer cages 6 with different thicknesses from each other.

The scintillation crystals 4 and the spacer cages 6 are combined therebetween, thereby keeping each scintillation crystal 4 aligned with the silicon photomultiplier, centred with respect to said axis of symmetry Y and spaced apart from the internal surface of the shield 22A.

Preferably, the two or more spacer cages 6 define an external geometrical surface equal for all of them and an internal geometrical surface different for each one of them.

The external geometrical surface is arranged to match with the internal surface of the shield 22A, and the internal geometrical surface is arranged to match with said lateral wall 4C of the respective scintillation crystal 4, thereby said spacer cage 6 and said scintillation crystal 4 are stuck in said inner space.

According to some preferred embodiments, the spacer cage 6 comprises a lattice structure composed of a network of beam elements which defines windows and/or openings. The lattice structure is suitable to maximize both robustness of the spacer cage 6 and the width of openings, to minimize the weight of the detecting apparatus 10.

On this regard, the beam elements are of the flat beam type, wherein the width of both vertical or horizontal beam element defines the thickness of the spacer cage which determines the spacing between scintillation crystal and shield.

Preferably, said spacer cage 6 is made of plastic material to minimize the interference with the signal of the radionuclides.

SiPM 5 is a technology which has developed over the last twenty years, but which only recently has reached comparable or better performance than the traditional technology with Traditional Photomultiplier Tube.

The technology of SiPM 5 can use silicon Geiger micro-sensors implanted in a silicon wafer. When a light photon hits the Geiger micro-sensor, it produces a cascade of electrons producing an analog signal which is transmitted by SiPM 5 to a processing unit.

According to some embodiments of the present invention, said analog signal is transmitted through an electrical connection comprised in said detecting device 1.

To allow the transmission of the analog signal from SiPM 5 to the processing unit, said electrical connection passes in the passage channel 8.

Advantageously, the presence of the passage channel 8 allows to minimize the perforations in the shielding shell 2, by implementing a channel substantially shaped like a slot and preferably extended transversely to the longitudinal symmetry axis Y.

Preferably, the passage channel 8 has a rectangular cross section with the length of a long side comprised between 1 mm and 10 mm, preferably 4 mm, and the length of a short side comprised between 1 mm and 3 mm, preferably 1.5 mm.

Alternatively, the passage channel 8 has a circular cross section with a diameter comprised between 1 mm and 10 mm, preferably between 2 mm and 5 mm, more preferably 2.5 mm.

With reference to SiPM technology, one of the biggest advantage is the reduced sizes, in particular, according to some embodiments of the invention, SiPM has a thickness comprised between 1 mm and 4 mm, preferably 2 mm.

Therefore, SiPM 5 has a preferably square or rectangular or circular shape and a ratio of sizes extended on the geometrical plane/thickness comprised between 8 mm and 15 mm, preferably about 10 mm.

Advantageously, the so reduced sizes of SiPM allow to confine the detection volume of the detecting apparatus 10 almost completely to that of the scintillation crystal 4 only, which is the detection active part.

The reduced detection volume eases the shielding thereof from natural background radiations, thus getting a good compromise between the thickness of the lead shielding shell 2 and the weight of the detecting apparatus 10, considering the fixed volume container 3 is housing a sample to be analysed.

According to some preferred embodiments of the invention, the detection volume comprises SiPM, the scintillation crystal 4, the fixed volume container 3 for a sample to be analysed and the spacer/aligner cage 6.

Preferably, the shielding shell 2, when the lid 2C is in closed position, the aligner cage 6 and the scintillation crystal 4 each have a cylindrical shape.

Thanks to the above-mentioned features, the detecting apparatus 10 can have a weight comprised between 8 Kg and 25 Kg, preferably between 10 Kg and 15 Kg, for example a weight of 11 Kg.

The detecting apparatus 10 also comprises a communication unit electrically connected to the processing unit.

The communication unit allows the data transmission from the detecting device 1 to an external receiver, allowing even an inexperienced operator to display the results obtained from the detecting apparatus 10 in terms of identified isotopes and radiation intensity.

It will be appreciated that said processing unit and said communication unit are preferably arranged outside said shielding shell 2, thus reducing the background radiations generated by the electronics.

Preferably, the communication unit comprises a Wi-Fi^{®} and/or Bluetooth^{®} transmitter.

According to some preferred embodiments of the invention, the processing unit implements a software for identifying the isotopes capable of determining the activity based on a pre-calibration of the absolute efficiency obtained through a Monte Carlo Code with data selected by a user or set by a guided procedure.

With reference to figures 3, 4A, 4B the detecting apparatus 10 comprises a casing 9 containing inside thereof the detecting device 1 and the processing and communication unit and wherein said top opening 22B is accessible through said casing 9 and wherein said casing 9 is closed when said lid 3 is in closed position.

Preferably, the detecting device 1 is arranged to receive emissions of electromagnetic radiations with energy comprised between 30 and 3000 KeV and in the energy range of 1460 keV, in case by using different scintillation crystals inside the detecting device 1.

Preferably, the scintillation crystal 4 is a crystal selected from the group consisting of: NaI(Tl)/LaBr₃:Ce, BeBr3:Ce.

Preferably, the detecting apparatus 10 has an energy resolution at Cs¹³⁷ less than 10%, preferably 7%.

Preferably, the detecting apparatus 10 has a photo-peak efficiency at Cs¹³⁷ of the cylindrical NaI(Tl) scintillation crystal 4 comprised between 18500 cpm/microSv/h and 19000 cpm/microSv/h, preferably 18840 cpm/microSv/h.

Under photo-peak efficiency the capability of the detector to measure the whole energy of a gamma photon is meant.

Preferably, the detecting apparatus 10 has a total efficiency at Cs¹³⁷ of the cylindrical NaI(Tl) scintillation crystal 4 comprised between 61500 cpm/microSv/h and 62000 cpm/microSv/h, preferably 61800 cpm/microSv/h.

Preferably, the detecting apparatus 10 has an uncertainty less than 5%.

Preferably, the detecting apparatus 10 allows to detect a Minimum Detectable Activity (MDA) of an air sample in the presence of an environmental radiation background higher than 200 nSv/h:
**MDA¹ Cs¹³⁷ < 7.5 Bq** Equivalent to < 1.8 Bq/m³ (assuming a pumping of 4 m³ of air)
**MDA¹ I¹³¹ < 4 Bq** Equivalent to < 1 Bq/m³ (assuming a pumping of 4 m³ of air)
**MDA² Cs¹³⁷ < 4 Bq** Equivalent to < 1 Bq/m³ (assuming a pumping of 4 m³ of air)
**MDA² I¹³¹ <2 Bq** Equivalent to < 0.5 Bq/m³ (assuming a pumping of 4 m³ of air)
¹ sizes of the fixed volume container for a sample to be analysed: with diameter of 50 mm x 27 mm.
² sizes of the fixed volume container for a sample to be analysed: with diameter of 50 mm x 1 mm.

The MDA value is calculated according to the following formula and according to the well known method described in "Glenn F Knoll - Radiation detection and measurement - John Wiley & Sons, 2010". $MDA = \frac{{N}_{D}}{{I}_{γ} t {ϵ}_{tot}} MDA expressed in Bq$
*I_{γ}* percentage of emission at a determined energy of an isotope
t time of measurement
*εₜₒₜ* absolute efficiency at the emission energy value of an isotope ${N}_{D} = 4.65 {σ}_{NB} + 2.71$ Where:
   *N_{D}* net count value measured in energy window;
   *σ_{NB}* sigma of the natural radiation background count detected in energy window;
   MDA is calculated at 95% of confidence level (5% of false negatives).

Preferably, the detecting apparatus 10 has an operating temperature comprised between -30°C and +60°C, preferably between -20°C and +50°C.

Under operating temperature, the temperature at which the detecting apparatus 10 keeps its technical features is meant.

With reference to figures 3, 4A and 4B, the casing 9 of the detecting apparatus 10 comprises a first handle 11.

The first handle 11 can be kinematically connected with the lid 2C of the shielding shell 2 so that by moving the first handle 11 said lid 2C passes from an open position to a closed position or vice versa.

According to some embodiments, the first handle 11 comprises two vertical elements 11A, substantially perpendicular to a resting plane of the detecting apparatus 10, and a transversal element 11B which puts in connection the two vertical elements.

The two vertical elements 11A are spaced apart from each other, thus making the motion of the lid 3 smoother in a space interposed between the same vertical elements.

Preferably, the two vertical elements 11A comprise guides 11C and the lid 2C comprises sliding elements arranged to slide in the guides 11C.

Moreover, the lid 2C can comprise a second handle 12 with second vertical elements 12A with height equal to half or one third with respect to the height of the vertical elements 11A of the first handle 11.

Advantageously, the first handle 11 and the second handle 12 allow an operator to grasp the second handle 12, to make it to slide along the guides 11C and to bring it near the transversal element 11B of the first handle 11.

In this way, it is possible to transport the detecting apparatus 10 with the lid 2C in open position, by grasping with one single hand the first handle 11 and the second handle 12.

Preferably, the first handle 11 swivels around a fulcrum passing through a base of said vertical elements 11A, in this way once opened said lid 2C and made it to slide along said guides 11C, it is possible to tilt the first handle 11 and rest the lid 2C on the casing 9 of the detecting apparatus 10.

In this way, an operator has both hands free while positioning the fixed volume container 3 containing the sample to be analysed.

With reference to figure 3, the detecting apparatus 10 comprises an activation button 13 also allowing inexperienced personnel to activate the detecting apparatus 10.

Preferably, the detecting apparatus 10 comprises a USB port 14 or the like which can be used both to monitor real-time data via cable, to download data and to upload data.

Preferably, the detecting apparatus 10 comprises a battery, alternatively it is connected to the electricity grid through said electric access door 14.

According to some embodiments, the detecting apparatus 10 has a width comprised between 150 mm and 200 mm, preferably between 160 mm and 170 mm, more preferably 165 mm, a depth comprised between 150 mm and 200 mm, preferably between 160 mm and 170 mm, more preferably 165 mm, a height comprised between 200 mm and 300 mm, preferably between 250 mm and 270 mm, more preferably 260 mm.

Advantageously, the reduced sizes and weight of the detecting apparatus 10 make it a transportable tool which allows the source analysis in situ, without having to move the sample to be analysed from the collection site to an analysis site.

To perform the identification of radioisotopes and to evaluate their concentration in situ, a method for monitoring the type and concentration of radionuclides in a fixed volume sample provides to arrange an apparatus 10 for detecting radiations from radionuclides comprising a shielding shell 2, a scintillation crystal 4, a silicon photomultiplier (SiPM) 5, a calibration unit and a communication unit.

The method provides the activation of the detecting apparatus 10 to acquire radiations generated by the environmental background and to perform a self-calibration of the detecting apparatus 10.

The self-calibration can take place by means of a software implemented on the processing unit which, through a Monte Carlo Code, evaluates the absolute efficiency by simulating the emission of radiation energies. Preferably, the simulated energies correspond to 25 in the energy range of interest.

Preferably, when possible, the simulated energies of the radiation are coincident with those of experimental interest and those typically used for calibrations. The detecting apparatus then results to be pre-calibrated in absolute efficiency for each type of sample declared in the design requirements.

The detecting apparatus 10 is further provided with absolute efficiency tables even for point sources for possible functionality checks by a specialized operator.

While performing the self-calibration of the detecting apparatus 10, the method provides to arrange a sample to be analysed in a container.

Preferably, said sample is a sample of air.

Preferably, to obtain said sample of air, between 2 m³ and 8 m³ of air are pumped in the container 3, more preferably 4 m³ of air are pumped in the container 3.

The time for pumping 4 m³ of air can be comprised between 15 min and 60 min, more preferably is 30 min and even the time for the self-calibration can be comprised between 15 min and 60 min, more preferably is 30 min.

Once the sample has been arranged inside the container 3, the latter is arranged in the detecting apparatus 10.

At this point, the detecting apparatus 10 starts to identify the isotopes which generate emissive gamma activity in the sample and to perform the measurement of the emissive gamma activity of the sample.

Preferably, the time for identifying the isotopes and measuring their concentration is comprised between 15 min and 60 min, more preferably 30 min.

Once the identification and the concentration measurement of the isotopes have been performed, the detecting apparatus 10 transmits the obtained data to an electronic device to make them visible to an operator.

At last, said method does not provide to have to transport said sample from one collection site to another place dedicated to the analysis of said sample.

This last feature is the main advantage of the method according to the invention, which allows to perform a measurement in situ, wherein the measurement has a quality comparable or higher than the measurements of laboratory devices and wherein the monitoring can be performed even by an inexperienced operator.

To the above-described monitoring method and detecting apparatus used for applying the method, a person skilled in the art, with the purpose of satisfying additional and contingent needs, could introduce several modifications and variants, however all comprised within the protective scope of the present invention, as defined by the enclosed claims.

## Claims

1. A detecting apparatus (10) for identifying and monitoring ionizing radiations, provided with a detecting device (1) wherein a longitudinal symmetry axis (Y) is defined, wherein the detecting device (1) in turn comprises:
* a shielding shell (2) for ionizing radiations, formed by a base (2B) and by a shield (22A) mechanically coupled so as to form an inner space and a top opening (22B) opposite the base (2B), the shielding shell (2) being provided with a sealing lid which is movable between an open position and a closed position wherein the top opening (22B) is hermetically sealed;
* a scintillation crystal (4), housed in said inner space, extending along said longitudinal symmetry axis (Y) and having a receiving base (4A) and a transmission base (4B) opposed and separated by at least one lateral wall (4C);
* a fixed volume container (3), apt to receive a sample to be analysed, contained in said shielding shell (2) in a position adjacent to the receiving base (4A) of the scintillation crystal (4) and at said top opening (22B);
* a photomultiplier (5), substantially extended on a geometrical plane adjacent to said transmission base (4B) of the scintillation crystal (4), provided with an electrical connection;
* a spacer cage (6) inserted in an empty space (2A) of said shielding shell (2) containing the scintillation crystal (4) surrounding said at least one lateral wall (4C) so as to keep the scintillation crystal (4) aligned with the silicon photomultiplier, centred with respect to said axis of symmetry (Y) and spaced apart from the internal surface of the shield (22A),
wherein, in the shielding shell (2), between the shield (22A) and the base (2B), a recess (8) is formed implementing a passage channel (8A) for said electrical connection; and
wherein said spacer cage (6) defines an external geometrical surface arranged to match with said internal surface of the shield (22A), and an internal geometrical surface arranged to match with said lateral wall (4C), thereby said spacer cage (6) and said scintillation crystal (4) are stuck in said inner space.

2. The detecting apparatus (10) according to claim 1, comprising a processing unit, connected to said electrical connection so as to receive an analog signal generated by the photomultiplier (5), electrically connected to a communication unit arranged for a data transmission from the detecting device (1) to an external receiver, wherein both the processing and the communication units are arranged externally to the shielding shell (2) of the detecting device (1).

3. The detecting apparatus (10) according to claim 1 or 2, wherein said container (3) comprises a cellulose filter.

4. The detecting apparatus (10) according to claim 3, wherein said container (3) comprises an active carbon filter, the container (3) being arranged to contain air samples.

5. The detecting apparatus (10) according to any one of the preceding claims, wherein the shield (22A) of said shielding shell (2), said aligner cage (6) and said scintillation crystal (4) all have a complementary cylindrical shape.

6. The detecting apparatus (10) according to any one of the preceding claims, wherein said detecting device (1) is arranged to receive emissions of electromagnetic radiations with an energy comprised between 30 and 3000 KeV and in the energy range of 1460 keV, in case by using different scintillation crystals (4) inside the detecting device (1).

7. The detecting apparatus (10) according to any one of the preceding claims comprising a casing (9), enclosing said detecting device (1) and said processing and communication units, wherein said top opening (22B) is accessible through said casing (9) and such access is arranged to be closed when said lid (2C) is in a closed position.

8. A kit for identifying and monitoring ionizing radiations comprising
- a detecting apparatus (10) according to one or more of claims 1 to 7;
- two or more scintillation crystals (4) with different sizes from each other;
- two or more spacer cages (6) with different thicknesses from each other, said two or more spacer cages (6) defining an external geometrical surface same for all of them and an internal geometrical surface different for each one of them;
wherein said scintillation crystals (4) and said spacer cages (6) are combined therebetween so as to keep aligned each scintillation crystal (4) with the silicon photomultiplier, centred with respect to said axis of symmetry (Y) and spaced apart from the internal surface of the shield (22A) and wherein said external geometrical surface is arranged to match with said internal surface of the shield (22A), and said internal geometrical surface of each spacer cage (6) is arranged to match with said lateral wall (4C) of the respective scintillation crystal (4), thereby said spacer cage (6) and said scintillation crystal (4) are stuck in said inner space.

9. A method for monitoring the type and concentration of radionuclides in a fixed volume sample, comprising the following steps:
• arranging a detecting apparatus (10) according to any one of the preceding claims;
• acquiring radiations generated by the environmental background;
• performing a self-calibration of the detecting apparatus (10); and
• arranging a sample to be analysed in a container (3);
• arranging said container (3) in said detecting apparatus (10);
• identifying the isotopes which generate emissive gamma activity in the sample;
• performing the measurement of the emissive gamma activity of the sample; and
• transmitting the obtained data to an electronic device to make them visible to an operator.

10. The monitoring method according to claim 9, wherein said self-calibration takes place by means of a software implemented on the processing unit which through a Monte Carlo Code evaluates an absolute efficiency of said detecting apparatus (10) by simulating the emission of energies of the radiation.
